# EUROPEAN PATENT SPECIFICATION

(11) **EP 4 547 644 B1**
(45) Date of publication and mention of the grant of the patent: **05.08.2026**
(21) Application number: 23733734.0
(22) Date of filing: 19.06.2023
(51) Int. Cl.: C07C 209/84, C07C 209/16, C07C 211/10

(54) **METHOD FOR MANUFACTURE OF ETHYLENEAMINES**
VERFAHREN ZUM HERSTELLEN VON ÄTHYLENAMINEN
PROCÉDÉ DE FABRICATION D'ÉTHYLÈNEAMINES

(30) Priority: 29.06.2022 EP 22181909
(43) Date of publication of application: 07.05.2025
(73) Proprietor: BASF SE, 67056 Ludwigshafen am Rhein (DE)
(72) Inventor: LUYKEN, Hermann, 67056 Ludwigshafen am Rhein (DE); KRUG, Thomas, 67056 Ludwigshafen am Rhein (DE); KOENEMANN, Martin, 67056 Ludwigshafen am Rhein (DE); JAEGLI, Stephanie, 67056 Ludwigshafen am Rhein (DE); HEIDEMANN, Thomas, 67056 Ludwigshafen am Rhein (DE); BECKER, Barbara, 67056 Ludwigshafen am Rhein (DE); JOEDECKE, Michael, 67056 Ludwigshafen am Rhein (DE)
(74) Representative: BASF IP Association
(86) International application number: PCT/EP2023/066376
(87) International publication number: WO 2024/002740

(56) References cited:
- US-A- 4 032 411
- US-A1- 2017 217 874
- US-A1- 2020 308 098

## Description

The present invention relates to a process for the manufacture of ethylenediamine (EDA).

Ethylenediamine is used predominantly as an intermediate for the production of bleach activators, crop protection agents, pharmaceuticals, lubricants, textile resins, polyamides, paper auxiliaries, gasoline additives and many other substances.

There are numerous known processes for preparing EDA (see, for example, Ullmann's Encyclopedia of Industrial Chemistry, "Amines Aliphatic", section 8.1.1. DOl: 10.1002/1436007.2a02_001.pub2).

In the preparation of ethylenediamine, N-methylethylenediamine (NMEDA) can be formed by side reactions. For example, in the reaction of monoethanolamine (MEA) with ammonia to give EDA, a degradation reaction of monoethanolamine can directly give rise to carbon monoxide (CO) and methylamine (decarbonylation). The methylamine can in turn react directly with further monoethanolamine to give NMEDA.

NMFDA can also form in the dimerization of monoethanolamine to aminoethanolamine (AEEA) when AEEA is degraded directly by decarbonylation to NMEDA.

NMEDA can also form in the preparation of EDA from C1 units such as hydrogen cyanide and formaldehyde.

Besides NMEDA, other poly-N-methylated ethylenediamines can also form, for example bis(N-methyl-1,2-ethanediamine). In terms of amount, however, the formation of NMEDA is typically dominant.

For most industrial applications, the market demands a purity for EDA of at least 99.5% by weight. Organic secondary components, including NMEDA, may be present with a proportion of not more than 0.5% by weight. Furthermore, the water content may be not more than 0.5% by weight. More particularly, in many industrial applications, a purity of EDA is specified where the proportion of NMEDA is below 1000 ppm by weight.

EDA which, as a result of its preparation, has a higher water and/or NMEDA content has to be worked up correspondingly, so as to obtain EDA that has the required specifications.

A challenge encountered in the separation of ethyleneamine mixtures is that EDA and water as well as NMEDA and water form azeotropes. Azeotropic mixtures cannot be separated by conventional distillation. On the other hand, the formation of the azeotropic hydrates of EDA and NMEDA may enhance the boiling point differences between NMEDA and EDA, making separation of NMEDA and EDA less difficult under conditions under which their corresponding hydrates form.

Depending on the amount of NMEDA in the ethylene diamine mixture, different distillation strategies may be employed for ethylene amine mixtures comprising NMEDA, EDA and water.

If the ethylenediamine mixture comprises high amounts of NMEDA, NMEDA is usually separated before water is removed.

EP2487151 (DOW) discloses a process for depleting alkylethyleneamines from ethyleneamine mixtures, wherein a mixture consisting of ethylenediamine, water and one or more alkylethylenediamines is subjected to such conditions that an azeotrope is formed between the water and the alkylethyleneamines and the azeotrope of water and NMEDA is separated from the remaining composition. It is disclosed that the pressure in the rectification column in which the azeotrope of water and alkylethylenediamine is separated is in the range from 1.01 to 2.12 bar, preferably 1.5 to 1.98 bar. In example 1, the distillation is effected at a top pressure of 1.634 bar, a top temperature of 115°C and a bottom temperature of 176°C. Apart from these technical details relating to the distillation, the disclosure does not seem to contain any further technical information as to which measures the person skilled in the art has to take into account so that an azeotrope of alkylethyleneamine and water is formed.

A further process for separating NMEDA from EDA and water is disclosed in EP2507202 (BASF). This disclosure teaches that the removal of NMEDA is effected in a rectification column at a column top pressure in the range from 0.01 bar to 4 bar and that the mixture to be distilled comprises at least a sufficient amount of water that the condition H = a*X/Y is fulfilled, where H is the proportion by weight of water in the mixture to be distilled, X is the proportion by weight of water and Y is the proportion by weight of EDA at the azeotropic point of a binary mixture of water and EDA at the column pressure in question, and a is a real number having a value of 0.9 or more.

In WO 2019/081284 (BASF), a process for the separation of NMEDA from EDA and water is disclosed where the NMEDA-separation column is operated at a bottom temperature of 155°C and less and where the NMEDA-separation column comprises 50 to 140 theoretical stages. NMEDA is drawn at the top of the column and the azeotropic mixture of EDA/water is drawn at the bottom of the column.

After the removal of NMEDA or if the NMEDA concentration is low from the beginning, the EDA/water mixture can be separated in different ways.

DE 1258413(DOW) discloses the separation of EDA and water in a single dehydration column which is operated at pressures at which the azeotrope between water and EDA is broken, so that water can be drawn at the top of the distillation column and EDA and other amines are drawn from the sump.

Alternatively, EDA and water may be separated in two columns operated at different pressures (dual pressure distillation or pressure swing distillation) (see Fulgueras, A.M., Poudel, J., Kim, D.S. et al. Korean J. Chem. Eng. (2016) 33: 46. https://doi.org/10.1007/s11814-015-0100-4).

Several patent applications (CN105585508 (Sinopec), CN105585508 (Sinopec), CN105585501 (Sinopec), CN105585501 (Sinopec), CN104119297 (Xi'an Modern Chemistry Research Institute), CN104230850 (Sinopec), CN105523943 (Sinopec)) teach the separation of water and ethylene diamine using entraining agents which form a low boiling azeotrope with water, such as toluene or xylene.

US3055809 (Jefferson Chemicals) discloses the distillation of an EDA/water mixture under azeotropic conditions. The water/EDA mixture is fed to the middle of a rectification column. A high boiling extraction solvent is fed to the top of the distillation column where it flows in a counter current to the rising azeotropic EDA/water vapors. Through the contact of the EDA/water with the high boiling extraction agent, EDA is essentially enriched in the extraction solvent so that essentially pure water is obtained at the top of the column and a water depleted mixture of EDA, the extraction solvent and water is obtained at the bottom of the column. According to the invention, suitable extraction solvents are solvents with a boiling point above 120°C, such as polyhydric alcohols, including the glycols, such as ethylene glycol (MEG), propylene glycol and butylene glycols and the glycerols, such as glycerine. Other effective solvents are the hydroxyamines or alkanolamines, such as monoethanolamine (MEOA), diethanolamine (DEOA), triethanolamine (TEOA) and propanolamine.

WO2021/115907 (BASF) discloses the distillation of NMEDA, water and EDA in a single distillation column at pressures where the azeotrope between EDA and water is broken. In a narrow pressure range, the separation of NMEDA, EDA and water can be conducted in a single column.

US2017217874 relates to a process for purifying ethylenediamine (EDA) by distillation, which involves feeding a mixture of water, EDA, and N-methylethylenediamine (N-MeEDA) into a distillation apparatus, separating water overhead at a pressure greater than 4.8 bara, and then distilling off N-MeEDA from the water-depleted mixture in a second distillation apparatus, to obtain EDA with reduced water and N-MeEDA content. The process may further include removing ammonia and diethylenetriamine (DETA

In US4032411 (Berol Kemi AG) a water/EDA mixture is distilled in the presence of a distillation adjuvant. The distillation adjuvant is described to act as an azeotrope breaker. Accordingly, in a mixture of water, EDA and the distillation adjuvant the azeotrope between water and EDA is broken so that water can be removed overhead, and a mixture of EDA and the distillation adjuvant can be removed at the bottom of the distillation column. Following compounds are disclosed to be suitable distillation adjuvants: PIP, DETA, AEEA, AEP and mixtures thereof. The distillation is conducted at around 1 to 3 bar with temperatures at the bottom of the distillation column varying between 140 to 210°C. According to the invention, in order for the distillation to be carried out under technically appropriate conditions to permit the removal of water from EDA without the formation of an azeotrope, the weight ratio between the distillation adjuvant and EDA should be within the range of about 2:8 to 9:1, preferably from about 4:6 to 8:2. According to the invention, when preparing EDA having a very low water content of below 2% by weight, it is preferred to carry out the distillation in more than one distillation column, because otherwise very high distillation temperatures would be required, which could lead to a decomposition of the amino compounds and the column would require a large number of theoretical stages. Accordingly, a preferred embodiment of the invention of US4032411 comprises the steps of firstly removing the major proportion of water present in the aqueous EDA-solution by carrying out the distillation in the presence of one or more adjuvants, followed by removing the adjuvant and finally carrying out a vacuum distillation to remove additional water.

Depending on the composition of the ethyleneamine mixture, the separation of the components to obtain an in-spec EDA can be challenging. When distilling under azeotropic conditions to remove NMEDA, the ratio of water to NMEDA and EDA needs to be controlled to achieve azeotropic conditions. Due to the small differences in the boiling point between NMEDA and EDA and their corresponding azeotropes, distillation columns with many trays may be needed. Using entraining agents, distillation adjuvants or extraction solvents may require addition of an additional component, which may need to be removed from the final products. Distilling under non-azeotropic conditions without the addition of entraining agents, adjuvants or extraction solvents usually requires high operating pressures of the distillation column.

Accordingly, there is continuous need for a process allowing for the manufacture of in-spec EDA using an economic distillation process having a reasonable number of columns and in which the columns may be operated with a reasonable number of trays and at reasonable temperatures and pressures. There is also continuous need for a process in which components are avoided which are not produced or converted in the process itself. The use of such additional components requires a larger dimensioning of equipment to handle the additional components and the additional components usually need to be separated in an additional step to allow recycling of such adjuvants to the process. The object of the present invention was therefore to provide a process for the production of EDA using a reasonable number of distillation columns, reasonably dimensioned of columns and operating such columns at reasonable conditions, such as temperature and pressure, wherein reasonably with respect to the foregoing means finding a suitable balance between operating expenditures, capital expenditures and product quality.

The object of the present invention was achieved by
a method for the manufacture of ethyleneamines from a mixture comprising water (H₂O), ethylenediamine (EDA) and N-methylethylenediamine (NMEDA), comprising the steps of:
(i) providing a feed stream comprising EDA, NMEDA and water;
(ii) separating the feed stream provided in step (i) in the one or more distillation columns into
   a. a fraction A comprising water and NMEDA wherein the weight ratio of water to NMEDA in fraction A is more than 100:1;
   b. a fraction B comprising water, NMEDA and EDA wherein the weight ratio of water to NMEDA is in the range of 1:100 to 100:1; and
   c. a fraction C comprising water and EDA wherein the weight ratio of EDA to water is more than 5:1, and wherein the water content in fraction C is in the range of 0.1 to 10 percent by weight;
(iii) separating fraction C into:
   - a fraction C-1 comprising EDA, NMEDA and water, wherein the weight ratio of EDA to water is in the range of 1:5 to 5:1 and the weight ratio of NMEDA to EDA is in the range of 100:1 to 1:1, and
   - a fraction C-2 comprising EDA and preferably components with a boiling point higher than EDA and in which the NMEDA content is 0.01 percent by weight or less.

Surprisingly it was found that when separating the feed into the fractions according to the invention, the downstream separation steps can be tailored to allow for an economic separation of the feed into the desired value components and to obtain the desired value components in a high quality. Further, it was found that separating the feed into the inventive fractions, an efficient recycling of materials can be achieved.

The following abbreviations are used herein:
- AEEA:: aminoethylethanolamine
- AEP:: aminoethylpiperazine
- DEOA:: diethanolamine
- DETA:: diethylenetriamine
- EDA:: ethylenediamine
- EDC:: ethylene dichloride
- HEDETA:: hydroxyethyldiethylentriamine
- HEPIP:: hydroxyethylpiperazine
- HPA:: heavy polyamines
- MEOA:: monoethanolamine
- MEG:: monoethylene glycol
- NMEDA:: N-methylethylenediamine
- PEHA:: pentaethylenehexamine
- PIP:: piperazine
- TEPA:: tetraethylenepentamine
- TETA:: triethylenetetramine

Unless specified otherwise, pressure figures relate to the absolute pressure figure.

The term ethyleneamines used herein comprises ethylenediamine (EDA) and its linear homologues of general formula (I):

R-CH₂-CH₂-NH₂ (I)

where R is a radical of the formula -(NH-CH₂-CH₂)ₓ-NH₂ where x is an integer in the range from 1 to 4, preferably 1 to 3 and most preferably 1 to 2. Preferably, the reaction output comprises DETA, TETA and TEPA, more preferably DETA and TETA and especially preferably DETA;
and cyclic components of the general formula (II): 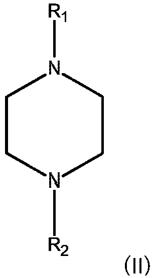
where R₁ and R₂ are independently or simultaneously either H or a radical of the formula - (CH₂-CH₂-NH)ₓ-CH₂-CH₂-NH₂ where X is an integer **in** the range from 0 to 4, preferably 0 to 4 and more preferably 1 to 2.

Examples of linear ethyleneamines are DETA, TETA, TEPA and HEPA.

Examples of cyclic ethyleneamines are PIP and AEPIP.

The term ethanolamines used herein comprises monoethanolamine (MEOA) and its linear homologues of general formula (III):

R-CH₂-CH₂-OH (III)

where R is a radical of the formula -(NH-CH₂-CH₂)ₓ-NH₂ where x is an integer in the range from 1 to 4, preferably 1 to 3 and most preferably 1 to 2.

### Examples of higher linear ethanolamines are AEEA, HEDETA

The tern ethanolamine as used herein comprises also cyclic ethanolamines of the formula (IV) 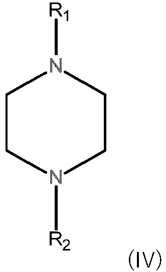 where R₁ is a radical of the formula -(CH₂-CH₂-NH)ₓ-CH₂-CH₂-OH where x is an integer in the range from 0 to 4, preferably 0 to 3 and more preferably 1 to 2, and R₂ is independently or simultaneously either H or a radical of the formula -(CH₂-CH₂-NH)ₓ-CH₂-CH₂-OH where x is an integer in the range from 0 to 4, preferably 0 to 3 and more preferably 1 to 2, or a radical of the formula -(CH₂-CH₂-NH)ₓ-CH₂-CH₂-NH₂ where x is an integer in the range from 0 to 4, preferably 0 to 3 and more preferably 1 to 2.

One example of a cyclic ethanolamine is hydroxyethylpiperazine (HEPIP).

### Step (i): Providing a feed stream comprising EDA, NMEDA and water:

The method according to the invention comprises the step (i) of providing a feed stream comprising EDA, NMEDA and water.

In a preferred embodiment, providing a feed comprising EDA, NMEDA and water in step (i) comprises the steps of:
(i-a) carrying out an EDA preparation process to obtain an output comprising EDA, NMEDA and water; and
(i-b) removal of ammonia and/or hydrogen from the output produced in step (i-a); and
(i-c) optionally adding an additional adjuvant.

### Step (i-a): EDA preparation process:

The feed provided in step (i) is preferably provided by carrying out an EDA preparation process.

The EDA preparation process of step (i-a) can be any known process for the manufacture of EDA, such as the MEOA process, the C1-process, the EDC process or the MEG process, as described below.

### MEOA process:

The reaction of MEOA and ammonia is described, for example, in US 2,861,995, DE-A-1 172 268 and US 3,112,318. An overview of the various process variants of the reaction of MEA with ammonia can be found, for example, in the PERP Report No. 138 "Alkyl-Amines", SRI International, 03/1981 (especially pages 81-99, 117).

The reaction of MEOA with ammonia is preferably conducted in a fixed bed reactor over a transition metal catalyst at 150-250 bar and 160-210°C or over a zeolite catalyst at 1-20 bar and 280-380°C.

Transition metal catalysts used with preference comprise Ni, Co, Cu, Ru, Re, Rh, Pd or Pt or a mixture of two or more of these metals on an oxidic support (e.g. Al₂O₃, TiO₂, ZrO₂, SiO₂). Preferred zeolite catalysts are mordenites, faujasites and chabazites.

To achieve a maximum EDA selectivity, in the case of transition metal catalysis, a molar ratio of ammonia to MEOA of 6-20, preferably 8-15, is generally employed, and, in the case of zeolite catalysis, generally 20-80, preferably 30-50.

The MEOA conversion is generally kept between 10% and 80%, preferably 40-60%.

In continuous operation, preferably, a catalyst space velocity in the range of 0.3-0.6 kg/(kg*h) (kg MEOA per kg cat. per hour) is established.

To maintain the catalyst activity, when metal catalysts are used, preference is given to additionally feeding 0.05-0.5% by weight (based on the MEOA +NH₃+H₂ reaction input) of hydrogen into the reactor.

### C1 process:

The EDA preparation process in step (i-a) can also be a C1 process in which formaldehyde, hydrogen cyanide, ammonia and hydrogen are converted to EDA.

For instance, US-A 2 519 803 describes a process for preparing EDA by the hydrogenation of a partly purified aqueous reaction mixture which results from an amination of formaldehyde cyanohydrin (FACH) and comprises aminoacetonitrile as intermediate. Formaldehyde cyanohydrin can in turn be obtained by reaction of formaldehyde with hydrogen cyanide. A process description for preparation of FACH can be found, for example, in application PCT/EP2008/052337, page 26, and in application WO-A1-2008/104582, page 30 (variants A) and B)), to which reference is made explicitly here.

DE-A 1154 121 relates to a further process for preparing EDA, wherein the hydrogen cyanide, formaldehyde, ammonia and hydrogen reactants are reacted in the presence of a catalyst in a "one-pot" process.

WO-A1-2008/104592 relates to a process for preparing EDA by hydrogenation of aminoacetonitrile. Aminoacetonitrile is typically obtained by reaction of formaldehyde cyanohydrin with ammonia, where formaldehyde cyanohydrin is in turn generally prepared from hydrogen cyanide and ammonia.

Preferably, a reaction output comprising EDA and NMEDA is prepared by the process described in WO-A-2008/104592, to which reference is hereby explicitly made.

### EDC process:

EDA can also be prepared by reaction of ethylene dichloride with ammonia (EDC process). The reaction of EDC with ammonia is described, for example, in EP 2346809, in the above-mentioned PERP Report and in the references cited therein.

### MEG process

In an especially preferred embodiment, the feed to be provided in step (i) is obtained by conversion of MEG with ammonia in the presence of an amination catalyst and hydrogen. The reaction of MEG with ammonia can be carried-out in the liquid phase or the gas phase. Gas phase reactions are disclosed, for example, in CN 102190588 and CN 102233272. Preferably the conversion of MEG with ammonia is conducted in the liquid phase according to US 4,111,840, US 3,137,730, DE 1 72 268, WO 2007/093514, WO2007/093552, WO2018/224316, WO2018/224315, WO2018/224321, WO2019/081283, WO 2019/081285 and WO2020/17085, to which reference is hereby explicitly made.

In a particularly preferred embodiment (MEG Process) the feed to be provided in step (i) is obtained by conversion of MEG with ammonia in the presence of an amination catalyst and hydrogen.

The MEG used in the process can be prepared from ethylene obtainable from petrochemical processes. For instance, in general, ethylene is oxidized in a first stage to ethylene oxide, which is subsequently reacted with water to give MEG.

The ethylene oxide can alternatively be reacted with carbon dioxide in what is called the omega process to give ethylene carbonate, which can then be hydrolyzed with water to give MEG. The omega process features a higher selectivity for MEG since fewer by-products, such as di- and triethylene glycol, are formed.

Ethylene used in the preparation of MEG can alternatively be prepared from renewable raw materials. For instance, ethylene can be formed by dehydration from bioethanol.

MEG can also be prepared via the synthesis gas route, for example by oxidative carbonylation of methanol to give dimethyl oxalate and subsequent hydrogenation thereof. Thus, a further possible petrochemical raw material for the preparation of MEG is also natural gas or coal.

Also, MEG may be used, which is obtained from the recycling of PET by various methods, such as glycolysis, methanolysis, hydrolysis, saponification and pyrolysis.

In the MEG Process, MEG and optionally additional MEOA is reacted with ammonia. Although additional MEOA may be added as an additional educt, it is preferred to conduct the MEG Process without additional MEOA.

The ammonia used may be conventional commercially available ammonia, for example ammonia with a content of more than 98% by weight of ammonia, preferably more than 99% by weight of ammonia, preferably more than 99.5% by weight, in particular more than 99.8% by weight of ammonia.

The MEG Process is preferably conducted in the presence of hydrogen.

The hydrogen is generally used in technical grade purity. The hydrogen can also be used in the form of a hydrogen-comprising gas, i.e., with additions of other inert gases, such as nitrogen, helium, neon, argon or carbon dioxide. Hydrogen-comprising gases used may, for example, be reformer off-gases, refinery gases etc., if and as long as these gases do not comprise any catalyst poisons for the catalysts used, for example sulphur components such as H₂S or CO However, preference is given to using pure hydrogen or essentially pure hydrogen in the process, for example hydrogen having a content of more than 99% by weight of hydrogen, preferably more than 99.9% by weight of hydrogen, more preferably more than 99.99% by weight of hydrogen, especially more than 99.999% by weight of hydrogen.

In the MEG Process, MEG is preferably reacted with ammonia and an amination catalyst in the liquid phase.

Preferred amination catalysts for the MEG Process are:
- the nickel-rhenium catalysts disclosed in US4,111,840,
- the nickel-copper catalysts disclosed in US 3,137,730,
- the ruthenium-cobalt catalysts disclosed in WO2007093514,
- the catalysts comprising cobalt, ruthenium and tin disclosed in WO2018224316,
- the catalysts comprising tin and a further active metal disclosed in WO2018224315,
- the impregnated catalysts disclosed in WO2018224321, or
- the rhenium comprising catalysts disclosed in WO202017085,
to which reference is hereby explicitly made. Special preference is given to the catalysts disclosed in the examples of the above-referenced documents.

In the present context, "reaction in the liquid phase" means that the reaction conditions, such as pressure and temperature, are adjusted such that ethylene glycol is present in the liquid phase and flows around the amination catalyst in liquid form.

The reaction of MEG and/or MEOA with ammonia can be conducted continuously or batchwise. Preference is given to a continuous reaction.

Suitable reactors for the reaction in the liquid phase are generally tubular reactors. The catalyst may be arranged as a moving bed or fixed bed in the tubular reactors.

Particular preference is given to reacting MEG and/or MEOA with NH₃ in a tubular reactor in which the amination catalyst is arranged in the form of a fixed bed.

If the catalyst is arranged in the form of a fixed bed, it may be advantageous, for the selectivity of the reaction, to "dilute", so to speak, the catalysts in the reactor by mixing them with inert random packings. The proportion of the random packings in such catalyst preparations may be 20 to 80, preferably 30 to 60 and more preferably 40 to 50 parts by volume. Preferably, the catalyst is not diluted.

Alternatively, the reaction is advantageously carried-out in a shell and tube reactor or in a single-stream plant. In a single-stream plant, the tubular reactor in which the reaction is carried-out may consist of a series connection of a plurality of (e.g. two or three) individual tubular reactors. A possible and advantageous option here is the intermediate introduction of feed (comprising the reactant and/or ammonia and/or H₂) and/or cycle gas and/or reactor output from a downstream reactor.

When working in the liquid phase, the MEG and/or MEOA plus ammonia are guided simultaneously in liquid phase, including hydrogen, over the catalyst, which is typically in a preferably externally heated fixed bed reactor, at pressures of generally 5 to 35 MPa (50-350 mbar), preferably 5 to 30 MPa, more preferably 15 to 28 MPa, and temperatures of generally 80 to 350°C, particularly 100 to 300°C, preferably 120 to 270°C, more preferably 130 to 250°C, especially 160 to 230°C.

The partial hydrogen pressure is preferably 0.25 to 20 MPa (2.5 to 200 bar), more preferably 0.5 to 15 MPa (5 to 150 bar), even more preferably 1 to 10 MPa (10 to 100 bar) and especially preferably 2 to 5 MPa (20 to 50 bar).

MEG and/or MEA and ammonia are supplied to the reactor preferably in liquid form and contacted in liquid form with the amination catalyst.

Either trickle mode or liquid-phase mode is possible.

It is advantageous to heat the reactants, preferably to the reaction temperature, even before they are supplied to the reaction vessel.

Ammonia is preferably used in 0.90 to 100 times the molar amount, especially in 1.0 to 20 times the molar amount, based in each case on the MEG or MEA used.

The catalyst hourly space velocity is generally in the range from 0.05 to 5.0, preferably 0.1 to , more preferably 0.2 to 1 kg (MEG+MEA) per kg of catalyst and hour.

In an especially preferred embodiment, the conversion of MEG to ethyleneamines, particularly EDA, and ethanolamines is incomplete so that unconverted MEG remains in the reactor effluent. MEG conversion rate is generally in the range of 5 to 80 %, preferably 10 to 70 %, more preferably 30 to 50 %. Incomplete conversion of MEG has the advantage that unconverted MEG can act as a distillation adjuvant which facilitates the inventive separation of EDA, NMEDA and water and as described below.

The conversion of MEG is generally carried-out in a manner so that the feed provided to step (ii) comprises MEG in an amount specified below. The degree of conversion can be adjusted by variation of operating parameters, such as the temperature of the reaction, the amount of added ammonia and the catalyst hourly space velocity. Decreasing the hourly space velocity usually increases the degree of conversion of MEG to MEOA and EDA.

### Combined MEG-MEOA Process:

In a preferred embodiment of this invention, the feed provided in step (i) is obtained by:
(i) converting ethylene glycol (MEG) with ammonia in the presence of an amination catalyst and hydrogen in a first reactor (MEG reactor) to obtain a reaction effluent comprising water, ethylenediamine (EDA), monoethanolamine (MEOA), unconverted MEG and other components having a boiling point higher than EDA and optionally NMEDA, and
(ii) separating MEOA from the reaction effluent obtained in (i), and
(iii) converting the MEOA separated in step (ii) with ammonia in the presence of an amination catalyst and hydrogen in a second reactor (MEOA reactor) to obtain a reaction effluent comprising water, ethylenediamine (EDA), unconverted monoethanolamine (MEOA) and other components having a boiling point higher than EDA and optionally NMEDA.

When MEOA in the reactor effluent of the MEG conversion is separated and converted in a separate reactor with a tailored catalyst under adjusted process conditions the overall yield and selectivity to EDA is improved. The MEOA-conversion step is preferably carried out under conditions described above for the MEOA-process.

The separation of one or more MEOA fractions can be carried out according to the separation sequence described below

In a more preferred embodiment, the MEG-MEOA-process comprises the additional steps of
(iv) separating MEG from the reaction effluent obtained in step (i); and
(v) recycling the MEG separated in step (iv) to step (i).

The separation of one or more MEG fractions can be carried out according to the separation sequence described below.

The reaction effluent of the MEG reactor and the effluent of the MEOA reactor are preferably combined in order to separate its components in a common work-up section. Using a common separation sequence has the advantage of reducing equipment costs and energy requirements.

Providing the feed in step (i) using a MEG Process and separating produced MEOA and converting separated MEOA in a subsequent MEOA process has the advantage that the overall selectivity and yield of EDA can be increased compared to producing the same amount of EDA in either a single MEOA or a single MEG reactor.

Operating an MEG reactor and a subsequent MEOA-reactor allows for the efficient recycling of unconverted MEOA.

### Step (i-b): Removal of ammonia and/or hydrogen from the output obtained in step (i-a):

The reaction effluents from the abovementioned preparation processes generally comprise ammonia and hydrogen.

Prior to feeding the output from these processes into separation step (ii), ammonia and/or hydrogen are preferably removed.

The amount of ammonia in the reaction outputs is typically in the range from 30% to 90% by weight, more preferably in the range from 40% to 85% by weight and most preferably in the range from 50% to 80% by weight.

The amount of hydrogen in the reaction effluents is preferably in the range from 0.01 to 20 percent by weight, more preferably 0.05 to 10 percent by weight and most preferably 0.1 to 5 percent by weight.

Hydrogen and ammonia can be separated from the reaction mixture by methods known to those skilled in the art.

In a particularly preferred embodiment, the removal of ammonia and/or hydrogen is conducted according to the process disclosed in WO2019081285 in the section titled "Ammoniakabtrennung - Stufe 2" (or "Ammonia Separation - Step 2" in English) or in WO2019/081283, also in the section titled "Ammoniakabtrennung - Stufe 2" (or "Ammonia Separation - Step 2" in English), to which reference is hereby explicitly made.

### Composition of the feed provided in step (i)

The feed provided from step (i) comprises NMEDA, water and EDA.

Preferably the feed comprises 0.001 to 1 percent by weight of NMEDA, more preferably 0.01 to 0.5 percent by weight and most preferably 0.02 to 0.1 percent by weight.

The amount of EDA in the feed is preferably 1 to 30 percent by weight, more preferably 3 to 25 percent by weight and most preferably 5 to 20 percent by weight.

Preferably the feed comprises 1 to 30 percent by weight of water, more preferably 2.5 to 25 percent by weight and most preferably 5 to 20 percent by weight.

The feed provided from step (i) preferably comprises 0.1 percent by weight of ammonia or less, more preferably 0.01 percent by weight or less and most preferably 0.001 percent by weight or less.

The feed provided to step (ii) preferably comprises 0.1 percent by weight or less of hydrogen, more preferably 0.01 percent by weight or less and most preferably 0.001 percent by weight or less.

The feed provided from step (i)preferably comprises other ethyleneamines and ethanolamines, especially ethyleneamines and ethanolamines having a boiling point higher than EDA, in particularly PIP, MEOA, DETA, TETA, AEP, HEPIP, AEEA and HEDETA. More preferably, the feed provided in step (i) comprises MEOA, preferably in an amount from 1 to 30 percent by weight, more preferably 3 to 25 percent by weight and most preferably 5 to 20 percent by weight.

Preferably the feed provided in step (i) comprises one more ethyleneamines or ethanolamines having a boiling point higher than EDA selected from the group consisting of PIP, DETA, AEPIP, HEPIP, AEEA, DEOA, TETA and HEDETA. The concentration of each selected ethyleneamine or ethanolamine is preferably in the range of 0.001 to 5 percent by weight, more preferably 0.01 to 3 percent by weight and more preferably 0.03 to 2.5 percent by weight.

When the feed to be provided in step (i) is obtained by the MEG Process described above, the feed (i) provided in step (i) preferably comprises 20 to 90 percent by weight of MEG, more preferably 25 to 80 percent by weight and more preferably 30 to 70 percent by weight of MEG.

When the feed provided in step (i) is obtained by the MEG Process, the feed preferably also comprises 1 to 50 percent by weight of MEOA, more preferably 3 to 30 percent by weight and more preferably 5 to 25 percent by weight of MEOA.

As set out above, the MEG content is preferably adjusted by the degree of conversion of MEG in the MEG process.

In an especially preferred embodiment, the feed provided **in** step (i) comprises:

| | |
|---|---|
| Water: | 10 to 20 percent by weight; |
| NMEDA: | 0,01 to 0,1 percent by weight; |
| EDA: | 10 to 25 percent by weight; |
| PIP: | 0,1 to 5 percent weight; |
| MEOA: | 10 to 20 percent by weight; |
| MEG: | 30 to 50 percent by weight; |
| DETA | 0.1 to 5 percent by weight; |
| AEPIP: | 0.05 to 0.5 percent by weight; |
| AEEA: | 0.1 to 5% percent by weight; |
| HEPIP: | 0.01 to 0.5 percent by weight; |
| DEOA: | 0.05 to 1 percent by weight; |
| TETA: | 0,01 to 2 percent by weight; |
| HEDETA: | 0.01 to 2 percent by weight; |
| Others: | 0.001 to 5 percent by weight. |

### Step (i-c): Optional addition of an additional adjuvant:

As described below, the separation step (ii) is preferably carried out under conditions, under which the formation of the azeotrope between EDA and water and/or NMEDA and water is broken, impaired or otherwise modified in a manner that the boiling points of NMEDA, EDA and water are in a range, so that water can be separated from EDA and/or NMEDA as a low boing fraction. As further described below, the formation of the azeotrope can be modified by carrying out the separation step (ii) under high pressures in which the azeotrope between EDA and water is broken, such as the pressures applied in DE 1258413 (DOW) or WO2021/115907 (BASF).

Alternatively, the formation of the azeotrope between EDA and water may be modified if the feed provided in step (i) comprises certain ethyleneamines and ethanolamines, in particular the adjuvants disclosed in In US4032411 (Berol Kemi AG) which are selected from the group consisting of PIP, DETA, AEEA, AEP and mixtures thereof.

Within the frame of the present invention, it has now been found that the formation of the azeotrope can efficiently broken, impaired or otherwise modified in the manner set out above, when the feed provided in step (i) comprises one or more additional adjuvants.

The term additional adjuvants, as used in the context of this invention, refers to adjuvants which are not ethyleneamines or ethanolamines, in particular, ethyleneamines and ethanolamines selected from the group consisting of PIP, DETA, AEEA, AEP and mixtures thereof as disclosed in US4032411 (Berol Kemi AG) are disclaimed as additional adjuvants.

The additional adjuvant is preferably a hydroxyl group comprising compound.

The additional adjuvant is more preferably a compound, or a mixture of compounds selected from the group consisting of:
(i) aliphatic monools,
(ii) aliphatic diols;
(iii) aliphatic triols; and
(iv) aliphatic tetrols.

Surprisingly, it has been found that the azeotrope between EDA and water and/or NMEDA and water is effectively modified by these additional adjuvants so that the separation step (ii) can be carried out in an especially effective manner, in particularly under ambient or moderate sub-atmospheric pressures, which are easier to realize than the high pressures to otherwise break the azeotrope disclosed in DE 1258413 (DOW) or WO2021/115907 (BASF).

Preferred aliphatic monools are linear or branched alcohols with one hydroxy group comprising 5 to 12 carbon atoms.

Particularly preferred aliphatic monools are 1-pentanol, 3-methyl-1-butanol, 2,2-dimethyl-1-propanol, cyclopentanol, 1-hexanol. Cyclohexanol. 1-heptanol, 1-octanol, 1-nonanol, 1-decanol, 1-undecanol and 1-dodecanol.

Preferred aliphatic diols are linear or branched aliphatic diols comprising 2 to 6 carbon atoms. Particularly, preferred aliphatic diols are ethylene glycol, 1,2 propylene glycol, 1,3 propylene glycol, 1,2-butanediol, 1,3-butanediol, 1,4 butanediol, 1,5 pentanediol and 1,6 hexanediol.

Further preferred diols are ethylene glycols, propylene glycols and butylene glycols consisting of 2 to 4 ethylene glycol, propylene glycol or butylene glycol units, such as diethylene glycol, triethylene glycol, di-1,2-propylene glycol, tri-1,2-propylene glycol, di-1,3-propylene glycol, tri-1,3 propylene glycol, di-1,4-butanediol, tri-1,4-butanediol, di-1,2-butanediol, tri-1,2-butanediol, di-1,3-butanediol and tri-1,3-butanediol.

Most preferred aliphatic diols are 1,2-diols, particularly ethylene glycol, 1,2-propylene glycol, and 1,2-butanediol. It is believed that compounds with vicinal hydroxyl groups can interact especially well with EDA to break or otherwise modify the azeotrope formed between EDA and water.

Preferred aliphatic triols comprise three to 6 carbon atoms. In particularly preferred aliphatic triols are glycerine and trimethyolpropane.

Preferred aliphatic tetrols comprise four to 6 carbon atoms. In particularly preferred aliphatic tetrols are pentaerythritol, erythritol or threitol.

More preferably, the additional adjuvants comprising one or more hydroxyl groups are compounds which have a boiling point higher than the boiling point of EDA or NMEDA, but which can still be separated from ethyleneamines and ethanolamines using reasonable conditions. This has the advantage that such adjuvants are not evaporated prior or together with the separation of NMDEA or EDA, so that their azeotrope modifying effect remains effective until NMDEA is effectively separated.

Also, it is preferred that the additional adjuvant is an aliphatic hydroxyl group-comprising compound to avoid discoloration.

Further, it is preferred that the additional adjuvant is miscible with the outputs obtained by the EDA preparation process.

The amount of additional adjuvant comprising one or more hydroxyl groups in the feed provided in step (i) is preferably adjusted in a manner so that the molar ratio of all hydroxyl groups (n(OH)) present in the feed to the amount of EDA (n(OH):n(EDA)) is 0.7:1 or more, preferably 1:1 or more, more preferably 1.5:1 or more, even more preferably 2:1 or more and most preferably 4:1 or more.

If the adjuvant is an aliphatic diol, in particularly a 1,2-diol such as MEG, the molar ratio of diol to EDA is preferably 1:1 or more, more preferably 1.2:1 or more, even mor preferably 1.5:1 or more and most preferably 2:1 or more. The higher the ratio of OH-groups to the amine groups of EDA, the higher the azeotrope modifying effect.

The amount of additional adjuvant is preferably adjusted by determining the hydroxyl group concentration or the diol concentration and the EDA concentration in the feed and adding the additional adjuvant.

In case the additional adjuvant is not MEG, the amount of additional adjuvant is preferably adjusted by adding the additional adjuvant prior to the separation step (ii) and preferably after the removal of ammonia and/or hydrogen, so that the hydroxyl groups of the additional adjuvant are not aminated during the EDA preparation step (i-a).

When the additional adjuvant is MEG, the amount of additional adjuvant is preferably adjusted by the degree of conversion of MEG with ammonia in the MEG Process, as described above.

The most preferred additional adjuvants are 1,2 diols and triethylene glycol (TEG). Especially preferred 1,2-diols are MEG, 1,2 propylene glycol and 1,2 butylene glycol.

MEG has the advantage that it is also a raw material for the production of EDA.

When MEG is used as an additional adjuvant, the amount of additional adjuvant in the feed can be adjusted by controlling the conversion of MEG in its reactions with ammonia as set out above.

TEG has the advantage that is preferably used as a distillation adjuvant to separate DETA from MEOA. Using TEG has the advantage that TEG can not only be use as an adjuvant for DETA/MEOA separation but finds an additional use in the separation of NMEDA from EDA and water. Accordingly, only one adjuvant is required for both separations.

### Separation Step (ii):

The feed provided in step (i) is separated in step (ii) into:
a. a fraction A comprising water and NMEDA wherein the weight ratio of water to NMEDA in fraction A is more than 100:1;
b. a fraction B comprising water, NMEDA and EDA wherein the weight ratio of water to NMEDA is in the range of 1:100 to 100:1; and
c. a fraction C comprising water and EDA wherein the weight ratio of EDA to water is more than 5:1.

As described above, the separation step (ii) is preferably carried out under conditions, under which the formation of the azeotrope between EDA and water and/or NMEDA and water is broken, impaired or otherwise modified in a manner so that water can be separated from EDA and/or NMEDA as a low boiling fraction.

As one alternative, the formation of the azeotrope can be modified by carrying out the separation step (ii) under high pressures in which the azeotrope between EDA and water is broken, such as the pressures applied in DE 1258413 (DOW) or WO2021/115907 (BASF). Such pressures are preferably in the range of 4 bar or more, more preferably 4.5 bar or more and most preferably 5 bar or more. In an especially preferred embodiment, the separation step is carried out at pressures in the range of 5 to 7.5 bar, more preferably 5.1 to 7.0 bar, more preferably 5.2 to 6.5 bar and most preferably 5.5 to 6.0 bar.

Alternatively, the formation of the azeotrope between EDA and water may be modified if the feed provided in step (i) comprises certain ethyleneamines and ethanolamines, in particular the adjuvants disclosed in In US4032411 (Berol Kemi AG) which are selected from the group consisting of PIP, DETA, AEEA, AEP and mixtures thereof.

In a particularly preferred embodiment, the formation of the azeotrope is modified by providing a feed in step (i) which comprises one or more of additional adjuvants as specified above.

In a particularly preferred embodiment of the invention, the separation step (ii) is carried out at pressures lower than those which are ordinarily required to break the EDA-water azeotrope described in DE1258413 or WO2021/115907. This has the advantage that the operating and capital expenditures for a separation carried out under low to medium pressures are significantly lower than for a distillation carried out at high pressures.

Preferably, the separation step (ii) is carried out at a pressure of 4 bar or less, more preferably 3 bar or less, even more preferably 2 bar or less and most preferably 1.5 bar or less. More preferably the separation step (ii) is carried out at a pressure in the range of 50 mbar to 4 bar, more preferably 200 mbar to 3 bar and most preferably 670 mbar to 1.5 bar. Preferably, the pressure is kept in a range to that the bottom temperature of the first column, in which step (ii) is carried out, is 165°C or less, preferably 160° or less and most preferably 155°C or less.

### NMEDA Removal Column - One Column Set-Up

In a preferred embodiment, the separation in step (ii) is carried out in a one distillation column (NMEDA Removal Column) in which fraction A is drawn-off at the top, fraction B is drawn-off as a side fraction and fraction C is drawn-off at the bottom of the distillation column.

The separation step (ii) according to the invention can be carried-out in rectification apparatuses, such as tray columns, such as bubble-cap tray columns, sieve tray columns, dual flow tray columns, valve tray columns, baffle tray columns or columns having random packings or structured packings. Preference is given to using internals with a low pressure drop, such as structured packings, for example in the form of sheet metal packing such as Mellapak 250 Y or Montz Pak (B1-250 type). It is also possible for a packing with lower or elevated specific surface area to be present, or it is possible to use a fabric packing or a packing with another geometry such as Mellapak 252.Y. The advantages for the use of such internals are the low pressure drop and low specific liquid holdup compared to valve trays, for example. In a preferred embodiment, the NMEDA Removal Column is a dividing wall column.

The internals may be disposed in one or more beds.

Preferably, the rectification column comprises a structured packing, in particular Mellapak 250.

The design and layout of the NMEDA Removal Column is determined by the capacity which is to be produced. Typically, the NMEDA Removal Column has a diameter of 0.5 to 2.5 m, preferably 0.8 to 2 m and most preferably 1 to 1.5 m and a bed height of 5 to 20 m, preferably 7 to 15 m and most preferably 8 to 12 m.

The number of theoretical stages in the NMEDA Removal Column is generally in the range from 10 to 40, preferably 15 to 35 and more preferably 20 to 30.

The energy required for the separation of the feed provided in step (i) in the NMEDA Removal Column is typically introduced by an evaporator in the bottom of the column. This evaporator is typically a natural circulation evaporator or forced circulation evaporator. Alternatively, it is possible to use evaporators with a short residence time, such as falling-film evaporators, helical tube evaporators, wiped-film evaporators, or a short-path evaporator. The feed provided in step (i) comprising EDA, NMEDA and water is preferably introduced in a spatial region between 25% and 75% of the theoretical plates of the NMEDA Removal Column. For example, the feed may be introduced to the middle section of the column. Preferably, the feed is preferably introduced between 25 to 75% of the theoretical plates and more preferably between 30 to 70% of the theoretical plates. For examples, if the column has 25 theoretical stages, the feed is preferably introduced between stage 10 and 15.

The NMEDA Removal Column generally has a condenser which is generally operated at a temperature at which the predominant portion of fraction A is condensed at the corresponding top pressure.

In general, the operating temperature of the condenser is in the range from 10 to 150°C, preferably 50 to 140°C and more preferably 80 to 120°C.

Suitable condensers are condensers having cooling coils or helical tubes, jacketed tube condensers and shell and tube heat exchangers.

As previously stated, the top pressure of the NMEDA Removal Column is preferably 4 bar or less, more preferably 3 bar or less, even more preferably 2 bar or less and most preferably 1.5 bar or less. The preferred range of the top pressure in the NMEDA Removal Column is in the range of 50 mbar to 4 bar, more preferably 200 mbar to 3 bar and most preferably 670 mbar to 1.5 bar.

The NMEDA Removal Column can be operated at a bottom temperature of 80 to 220°C, more preferably 100 to 200°C and mor preferably 130 to 190°C.

In a preferred embodiment the bottom temperature of the NMEDA Removal Column is 190°C or less, preferably 165°C or less and most preferably 155°C or less. When the bottom temperature does not exceed such values, EDA losses in fractions A and B can be further reduced.

The energy required for the evaporation of the feed provided in step (i) in the NMEDA Removal Column is typically introduced by a reboiler in the bottom of the column. This reboiler is typically a natural circulation reboiler or forced circulation reboiler. Alternatively, it is possible to use reboilers with a short residence time, such as falling-film reboilers, helical tube reboilers, wiped-film reboilers, or short-path reboilers.

Fraction A, comprising water and NMEDA, wherein the weight ratio of water to NMEDA in fraction A is more than 100:1, is preferably drawn-off at the top of the NMEDA Removal Column. More preferably the weight ratio of water to NMEDA in fraction A is more than 500:1, even more preferably more than 1000:1 or more and most preferably more than 2000:1.

Preferably, fraction A comprises 0.1 percent or less by weight of NMEDA, more preferably 0.01 percent by weight or less and most preferably 0.001 percent by weight or less of NMEDA.

Preferably, fraction A comprises 0.01 percent or less by weight of EDA, more preferably 0.001 percent by weight or less and most preferably 0.0001 percent by weight or less of EDA.

Preferably, fraction A comprises 0.01 percent of less, more preferably 0.001 or less and most preferably 0.0001 or less of components having a boiling point higher than EDA. Fraction A may comprise some components with a boiling point lower than EDA- other than water-, such as methane or ammonia, each in an amount of 0.1 percent by weight or less, more preferably 0.08 percent by weight or less and most preferably 0.05 percent by weight or less.

Removing a fraction A, which has a low content of organics, allows for the efficient disposal of fraction A in a conventional water purification plant or the direct recycling into the process or other processes where water is required.

Fraction B, comprising water, NMEDA and EDA wherein the weight ratio of water to NMEDA is in the range of 1:100 to 100:1, preferably 1:50 to 50:1 and more preferably 1:10 to 10:1 and most preferably 1:3 to 3:1, is preferably drawn-off as a side stream from the NMEDA Removal Column. The amount of fraction B drawn-off as a side draw is comparatively small to avoid EDA losses, but large enough to remove substantially the entire amount of NMEDA present in the feed to the NMEDA Removal Column. Preferably the weight ratio of the weight of the feed stream to the NMEDA Removal Column to the weight of the side draw removed from the NMEDA removal column is in the range of 300:1 to 1500:1, more preferably 400:1 to 1200:1 and more preferably 600:1 to 1000:1

Fraction B is preferably withdrawn in an area between the middle of the column and the top of the column, preferably in an area between the 50 and 90 percent of the theoretical stages, more preferably 55 to 80 percent of the theoretical stages and most preferably 60 to 70 percent of the theoretical stages. Preferably, fraction B is withdrawn above the feed to the NMEDA Removal Column.

Fraction B typically also comprises some EDA, whereby the weight ratio of NMEDA to EDA is preferably 0.1:1 to 5:1, more preferably 1:1 to 4:1 and most preferably 1.5:1 to 3:1. Preferably, fraction B comprises 10 to 75 percent by weight of NMEDA, more preferably 20 to 70 and even more preferably 30 to 60 percent by weight of NMEDA.

Removal of fraction B as a side stream has the advantage that most of the NMEDA formed in the EDA preparation process may be removed in a small but concentrated stream. Fraction B is preferably sent to incineration allowing for the efficient disposal of the unwanted side product NMEDA whilst only sacrificing negligible amounts of the value product EDA.

In a preferred embodiment, fraction C which is preferably withdrawn from the bottom of the NMEDA Removal Column has a low water content, which usually meets sales specifications. In this embodiment, the weight ratio of EDA to water is more 100:1, preferably more than 200:1 and more preferably more than 300:1.

Fraction C preferably comprises 0.1 percent by weight or less of NMEDA, preferably 0.05 percent by weight or less and more preferably 0.01 percent by weight or less of NMEDA. Fraction C preferably comprises 1 to 30 percent by weight of EDA, preferably 5 to 25 percent by weight and more preferably 7.5 to 15 percent by weight.

In this embodiment, fraction C preferably comprises components having a boiling point higher than EDA, particularly:
1 to 50 percent by weight of MEOA, preferably 5 to 30 percent by weight and more preferably 10 to 20 percent by weight;
40 to 95 percent by weight of MEG, preferably 50 to 90 percent by weight and more preferably 60 to 80 percent by weight.

In this preferred embodiment, Fraction C preferably also comprises one or more of ethyleneamines or ethanolamines selected from the group consisting of PIP, DETA, AEEA, DEOA, HEPIP, HEDETA and TETA, preferably each in an amount of 0.01 to 2 percent by weight, more preferably 0.02 to 1 percent by weight and most preferably 0.03 to 0.7 percent by weight.

In a preferred embodiment, when MEG is used as the only additional adjuvant, the preferred composition of fraction C withdrawn at the bottom of the NMEDA Removal Column is:
MEG: 60 to 80 percent by weight,
MEOA: 10 to 20 percent by weight,
EDA: 5 to 15 percent by weight,
PIP: 0.5 to 2 percent by weight,
DETA: 0.1 to 2 percent by weight,
AEEA: 0.01 to 1 percent by weight,
DEOA: 0.01 to 0.5 percent by weight,
TETA: 0.1 to 2 percent by weight,
Water: 0.001 to 0.5 percent by weight,
Others: 0.001 to 5 percent by weight.

### NMEDA Removal Column - Two Column Set-Up

Instead of using a single NMEDA Removal Column, the separation step (ii) may be conducted in two NMEDA Removal Columns. In the first column, fraction A is preferably separated at the top the first NMEDA Removal Column and fractions B and C are jointly removed at the bottom of the first NMEDA Removal Column. Accordingly, joint fractions B and C are further separated in a second NMEDA Removal Column where preferably fraction B is removed at the top of the column and fraction C is withdrawn at the bottom of the column.

### Additional Dewatering column

In an especially preferred embodiment, fraction C which is preferably withdrawn from the bottom of the first or second NMEDA Removal Column has a higher water content.

The higher water content is preferably reduced in a subsequent separation step, most preferably in another distillation column (Residual Water Removal Column).

In this embodiment, the subsequent separation step does not only allow the further reduction of the water content, but it also enables a further reduction of the NMEDA-content, which allows to obtain EDA with still a lower NMDEA-content. This ultra-low NMDEA-content allows for a subsequent simplification in the further work-up of the ethyleneamine mixture by being able to separate EDA, PIP from higher boiling components, in particularly MEOA and MEG, in a single dividing wall column, as further set out below.

According to the invention, the water content in fraction C is in the range of 0.1 to 10 percent by weight and preferably 0.5 to 5 percent by weight.

In this especially preferred embodiment, fraction C preferably comprises components having a boiling point higher than EDA, particularly:
10 to 70 percent by weight of EDA, preferably 20 to 60 percent by weight and more preferably 35 to 55 percent by weight;
1 to 50 percent by weight of MEOA, preferably 5 to 30 percent by weight and more preferably 10 to 20 percent by weight;
5 to 60 percent by weight of MEG, preferably 10 to 50 percent by weight and more preferably 20 to 40 percent by weight.

In this especially preferred embodiment, Fraction C preferably also comprises one or more of ethyleneamines or ethanolamines selected from the group consisting of PIP, DETA, AEEA, HEDETA, HEPIP, DEOA and TETA, preferably each in an amount of 0.01 to 2 percent by weight, more preferably 0.02 to 1.5 percent by weight and most preferably 0.05 to 1 percent by weight.

In a preferred embodiment of this especially preferred embodiment, MEG is used as the only additional adjuvant.

In this case, the preferred composition of fraction C withdrawn at the bottom of the NMEDA Removal Column is:
MEG: 20 to 40 percent by weight,
MEOA: 10 to 25 percent by weight,
EDA: 30 to 60 percent by weight,
PIP: 0.5 to 5 percent by weight,
DETA: 0.1 to 3 percent by weight,
AEEA: 0.01 to 0.5 percent by weight,
DEOA: 0.01 to 0.5 percent by weight,
TETA: 0.1 to 2 percent by weight,
Water: 0.5 to 5 percent by weight,
Others: 0.001 to 5 percent by weight.

As specified above, the fraction C is additionally separated into:
- a fraction C-1 comprising EDA, NMEDA and water, wherein the weight ratio of EDA to water is in the range of 1:5 to 5:1, preferably 1:3 to 3:1 and more preferably 1:2 to 2:1 and the weight ratio of NMEDA to EDA is preferably in the range of 100:1 to 1:1, more preferably 50:1 to 5:1 and most preferably 30:1 to 10:14, and
- a fraction C-2 comprising EDA and preferably components with a boiling point higher than EDA and in which the NMEDA content is preferably 0.01 percent by weight or less, more preferably 0.001 percent by weight and most preferably 0.0001 percent by weight or less.

As previously stated above, when fraction C is further separated into a fraction C-1 and C-2, EDA with an ultra-low NMEDA content may be obtained.

In a preferred embodiment, Fraction C-1 is preferably directly recycled as a vapor phase to the bottom of the NMEDA Removal column without condensation. This has the advantage that the energy required for the separation at the second column is used in the first column, reducing the energy requirements at the reboiler of the first column.

The separation of fraction C into fractions C-1 and C-2 is preferably carried-out in a conventional distillation column (Residual Water Removal Column).

The Residual Water Removal Column is usually a tray column, a bubble-cap tray column, a sieve tray column, a dual flow tray column, a valve tray column, a baffle tray column, or a column having random packings or structured packings. Preference is given to using internals with a low pressure drop, such as structured packings, for example in the form of sheet metal packing such as Mellapak 250 Y or Montz Pak (B1-250 type). It is also possible for a packing with lower or elevated specific surface area to be present, or it is possible to use a fabric packing or a packing with another geometry such as Mellapak 252.Y. The advantages for the use of such internals are the low pressure drop and low specific liquid holdup compared to valve trays, for example.

The internals may be disposed in one or more beds.

Preferably, the Residual Water Removal Column comprises a structured packing, in particular Mellapak 250.

The number of theoretical stages in the Residual Water Removal Column is generally in the range from 15 to 70, preferably 20 to 50 and more preferably 30 to 40.

The energy required for the separation of the feed provided in step (i) in the Residual Water Removal Column is typically introduced by a reboiler at the bottom of the column. This reboiler is typically a natural circulation reboiler or forced circulation reboiler. Alternatively, it is possible to use reboilers with a short residence time, such as falling-film reboilers, helical tube reboilers, wiped-film reboilers, or a short-path reboiler.

Fraction C from the NMEDA Removal Column is preferably introduced at the top of the Residual Water Removal Column, so that preferably the Residual Water Removal Column is designed to have a stripping section only.

In a preferred embodiment, the Residual Water Removal Column does not have a condenser and the uncondensed vapor withdrawn at the top of the column is preferably recycled to the sump of the NMEDA Removal Column, preferably through a valve.

The top pressure of the Residual Water Removal Column is preferably 4 bar or less, more preferably 3 bar or less, even more preferably 2 bar or less and most preferably 1.5 bar or less. The preferred range of the top pressure in the Residual Water Removal Column is in the range of 100 mbar to 3 bar, more preferably 500 mbar to 2 bar and most preferably 800 mbar to 1.5 bar.

The Residual Water Removal Column is preferably operated at a bottom temperature of 140 to 230°C, more preferably 150 to 210°C and mor preferably 170 to 190°C.

The Residual Water Removal Column is preferably connected to an evaporator (reboiler) at the bottom of the column. This evaporator is typically a natural circulation evaporator or forced circulation evaporator. Alternatively, it is possible to use evaporators with a short residence time, such as falling-film evaporators, helical tube evaporators, wiped-film evaporators, or short-path evaporators.

In a preferred embodiment, wherein MEG is used as the only adjuvant, the composition of fraction C-2 withdrawn at the bottom of the Residual Water Removal Column comprises:
MEG: 20 to 60, preferably 30 to 50 percent by weight,
MEOA: 10 to 40, preferably 15 to 25 percent by weight,
EDA: 10 to 50, preferably 20 to 40 percent by weight,
PIP: 0.1 to 10, preferably 0.5 to 5 percent by weight,
DETA: 0.1 to 10, preferably 0.5 to 5 percent by weight,
AEEA: 0.1 to 10, preferably 0.5 to 5 percent by weight,
DEOA: 0.05 to 2, preferably 0.1 to 1 percent by weight,
TETA: 0.01 to 2, preferably 0.05 to 0.5 percent by weight,
Water: 0.001 to 0.5, preferably 0.01 to 0.1 percent by weight,
NMEDA: 0.005, preferably 0.0005 or less percent by weight.

### Downstream Separation and Recycling:

Fractions C or C-2 can be further separated in a sequence of downstream separation steps.

Downstream separation steps are preferably conducted in conventional or dividing wall columns.

In a particularly preferred separation sequence, fraction C-2 from the bottom of the Residual Water Removal Column is separated into:
- a fraction D-1 comprising EDA and
- a fraction D-2 comprising PIP and
- a fraction D-3, comprising components having a boiling point higher than PIP, in particularly MEOA, MEG, DETA, AEE, DEOA and TETA.

Preferably, the separation into fractions D-1, D-2 and D-3 is preferably conducted in a single dividing wall column. The dividing wall column may be designed according to the teaching of WO 2005/037769, which discloses a dividing wall column in which EDA and PIP can be separated from other higher boiling ethanolamines and ethylenediamines.

Fraction D-1 preferably comprises a water content of 0.1 percent by weight or less, preferably 0.08 weight percent or less and more preferably 0.05 weight percent or less.

Fraction D-1 preferably comprises a PIP content of 0.05 percent by weight or less, preferably 0.01 weight percent or less and more preferably 0.005 weight percent or less.

Fraction D-1 preferably comprises a NMEDA content of 0.05 percent by weight or less, preferably 0.001 weight percent or less and more preferably 0.0005 weight percent or less.

Fraction D-2 preferably comprises an EDA content of 0.3 weight percent of less, preferably 0.2 weight percent or less and more preferably 0.1 weight percent or less.

Fraction D-2 preferably comprises a water content of 0.05 weight percent of less, preferably 0.01 weight percent or less and more preferably 0.005 weight percent or less.

Fraction D-2 preferably comprises a NMEDA content of 0.01 weight percent of less, preferably 0.001 weight percent or less and more preferably 0.0001 weight percent or less.

Fraction D-2 preferably comprises an EDA content of 0.1 weight percent or less, more preferably 0.05 weight percent or less and most preferably 0.01 weight percent or less. This particularly preferred separation sequence in which fraction C-2 is separated in a single dividing wall column enables the preparation of EDA and PIP meeting sales specifications with an ultra-low NMEDA content, which reduces investment and operation costs compared to a conventional set up of an EDA/PIP separation column followed by the separation of EDA and PIP in a further column.

Fraction D-3 is preferably separated in one or more further separation steps into value fractions or fractions which can be easily recycled to an EDA preparation process. More preferably, fraction D-3 is separated into value fractions or fractions which can be provided to an additional MEOA process or MEOA reactor, as described above.

Fraction D-3 is preferably further separated into:
- a fraction E-1 comprising MEOA with a MEG content of preferably 2000 ppm or less, preferably 1500 ppm or less and more preferably 1000 ppm or less; and
- a fraction E-2 comprising components having a boiling point higher than MEOA, in particularly MEG, DETA, AEE, DEOA and TETA.

This separation step if preferably conducted in a conventional column.

Fraction E-1 is preferably fed to a MEOA-reactor, in which MEOA is converted to ethyleneamines and ethanolamines with ammonia in the presence of an amination catalyst and hydrogen as disclosed in a preceding section. The effluent of the MEOA-conversion reactor can be combined with the effluent of a MEG-conversion reactor and be separated using the same separation sequence and facility as also disclosed above.

Fraction E-2 is preferably separated into:
- a fraction F-1 comprising MEG and preferably 1000 ppm or less of MEOA, more preferably 800 ppm or less and most preferably 600 ppm or less, and
- a fraction F-2 comprising components having a boiling point higher than MEG, in particularly DETA, AEE, DEOA and TETA.

This separation step if preferably conducted in a conventional column.

Fraction F-1 is preferably recycled to a MEG-reactor, where MEG is converted with ammonia in the presence of hydrogen and an amination catalyst to the MEOA and ethyleneamines and ethanolamines.

Fraction F-2 is preferably separated into:
- a fraction G-1 comprising DETA, MEG and AEPIP, and
- a fraction G-2 comprising AEEA and HEPIP.

Fraction G-1 can be further separated into:
- a fraction H-1 comprising MEG,
- a fraction H-2 comprising DETA and AEPIP.

This separation step if preferably conducted in a conventional column.

Fraction H-2 is preferably further separated into:
- a faction I-1- comprising DETA, and
- a fraction I-2 comprising DETA and AEPIP.

Fraction I-1 can be used as a value fraction of DETA meeting sales specifications.

Fraction I-2 is preferably recycled to the column in which the separation of fraction G-1 is carried out.

Fraction G-2 can be further separated into:
- a fraction J-1 comprising AEEA and HEPIP,
- a fraction J-2 comprising AEEA, DEOA, TETA and HEDETA.

This separation step if preferably conducted in a conventional column.

Fraction J-1 is preferably recycled to a MEOA-reactor.

Fraction J-2 can be further separated into:
- a fraction K-1 comprising AEEA, and
- a fraction K-2 comprising DEOA, TETA and HEDETA.

Fraction K-1 can be used a value fraction for AEEA meeting usual sales specifications for AFFA.

Fraction K-2 can be used a mixed fraction of certain applications, or it can be further separated into fractions consisting essentially of DEOA, TETA and HEDETA.

When using one or more of the above-described separation steps, it is possible to separate the ethanolamines and ethyleneamines obtained in the EDA preparation process into fractions comprising a single value-product, such as AEEA, DETA, EDA and PIP or into fraction comprising mixtures of ethanolamines, which can be easily recycled into an additional MEOA process, especially into a reactor where MEOA and other alkananolamines are converted to ethyleneamines (MEOA-reactor). The process sequence described above focusses on obtaining a limited number of value products, such as AEEA, DETA, EDA and PIP, and fractions which can be recycled to the EDA preparation process. In this way, a limited number of separation steps is required to prevent loss through undesired side products or components.

In another preferred embodiment, fraction C is further separated without separating fraction C into additional fractions C-1 and C-2 into:
- a fraction D-1, comprising EDA,
- a fraction D-2, comprising EDA, PIP and MEOA, and
- a fraction D-3, comprising MEG and components with a boiling point higher than MEG, in particularly DETA, AEE, DEOA and TETA.

This separation step if preferably conducted in a conventional column, wherein fraction D-1 is withdrawn as a top product, fraction D-2 is withdrawn as a side draw and fraction D-3 is withdrawn as a bottom product.

Fraction D-1 essentially comprises EDA and a water content of preferably 0.2 percent by weight or less, more preferably 0.15 weight percent or less and most preferably 0.1 weight percent or less.

The NMEDA content preferably is 0.1 percent by weight or less, more preferably 0.08 weight percent or less and most preferably 0.05 weight percent or less.

Fraction D-2 preferably comprises 80 weight percent or more of MEOA, more preferably 85 weight percent or more and most preferably 90 weight percent or more of MEOA and about 1 to 5 percent by weight of each EDA and PIP.

Fraction D-2 is preferably further separated into:
- a fraction E-1 comprising EDA and PIP, and
- a fraction E-2 comprising MEOA.

This separation step if preferably conducted in a conventional column.

Fraction E-1 is preferably further separated into a fraction F-1 comprising EDA and a fraction F-2 comprising PIP.

Fraction E-2 is preferably recycled to an MEOA process step which is integrated into the process.

Fraction D-3 is preferably separated into:
- a fraction G-1 comprising MEG, and
- a fraction G-2 comprising DETA and component with a boiling point higher than DETA.

Fraction G-1 is preferably recycled to the MEG Process step or used a scrubbing liquid to wash out MEG in the ammonia/hydrogen removal step (i-b), as described above, before being recycled to the MEG process step.

Fraction G-2 is preferably separated into:
- a fraction H-1 comprising MEG and DETA, and
- a fraction H-2 comprising AEEA, DEOA and TETA.

This separation step if preferably conducted in a conventional column.

Fraction H-1 is preferably recycled to the MEG Process.

Fraction H-2, comprising the highest boiling components is preferably removed from the process and either send to a flare or separated in its individual components in further separation steps.

The separation sequence according to the present invention allows for the production of EDA having an in-spec NMEDA content.

The inventive separation step allows for the efficient removal of NMEDA and water requiring low operating and investment expenditures.

In a preferred embodiment, the removal of NMEDA and water can be conducted at ambient or slightly sub-atmospheric conditions, which are easier to implement that the high-pressure separation processes known in prior art.

The inventive separation allows for subsequent, downstream separation sequences, by which in-spec value fractions can be obtained.

The inventive separation also allows for the separation of MEOA and MEG which can be recycled to an EDA preparation process or reactor, either a MEG process or an MEOA process step.

The inventive separation step further allows to design an overall MEG process around it, which enables a high selectivity and yields of the desired value products, such as EDA, DETA, AEEA and PIP while minimizing loss of through undesired side products.

In a preferred embodiment, the separation of water and NMEDA to in-spec levels can be carried out in a single column, which is especially economic with respect to reducing capital expenditures.

In a further preferred embodiment, the water content in fraction C is adjusted to a higher level, which allows to remove residual water and NMEDA in a further separation step. When combining a NMEDA Removal Column with a Residual Water Removal Column ultra-low water and/or NMEDA contents in EDA can be obtained. Combining a NMEDA Removal Column with a Residual Water Removal Column also allows to conduct the subsequent separation of the value products EDA and PIP in a single dividing wall column, compared to a two-column set-up in which EDA and PIP are conventionally separated. The use of a dividing wall column has an additional positive effect on capital and operating expenditures.

The benefits of the invention are demonstrated by the following examples:

### Examples:

The examples are based on calculations performed using a process simulation model. The simulations have been performed using CHEMASIM^{®}. For the calculation of thermodynamic properties of pure components, like the vapor pressure, DIPPR correlations have been used. For the description of phase equilibria, the ideal gas law is used to describe the vapor phase and the NRTL excess Gibbs energy model is used for the description of the liquid phase. The parameters of the DIPPR correlations and the parameters of the NRTL model were adjusted to experimental data. For the components, for which no experimental data are available, the UNIFAC group contribution method was used for the description of the liquid phase in phase equilibria calculations. The distillation columns have been modelled and calculated using the equilibrium stage model. The employed simulation and thermodynamic property models have been adjusted to reproduce experimental and plant data with very good accuracy.

### Example 1:

A feed was prepared in a combined MEG/MEOA process.

After the removal of and hydrogen and ammonia the feed comprised:

| | |
|---|---|
| water: | 15,80 percent by weight |
| NMEDA: | 0.04 percent by weight |
| EDA: | 18.81 percent by weight |
| PIP: | 1.81 percent by weight |
| MEOA: | 16.67 percent by weight |
| MEG: | 42.07 percent by weight |
| DETA: | 1.96 percent by weight |
| AEPIP: | 0.21 percent by weight |
| AEEA: | 1.65 percent by weight |

The molar ratio of MEG to EDA in the feed stream is 2.17 to 1. The molar ratio of hydroxyl groups to EDA was approximate 6:1.

1725,72 kg/h of the feed stream were fed to the NMEDA Removal Column where it was separated into a fraction A, a fraction B and a fraction C.

The NMEDA-Removal Column was equipped with a reboiler and had 28 stages

The feed was introduced above stage 12 (counted from the bottom).

The bottom temperature was 159°C.

644,26 kg/h of fraction A comprising water and 100 weight ppm NMEDA (weight ratio of water to NMEDA: approx. 10000:1) was withdrawn from the top of the column and fed to a condenser operated at 100°C.

372,12 kg/h of the condensed stream were refluxed back to the top of the NMEDA Removal Column, and a stream of 272,13 kg/h was removed.

1,58 kg/h of a fraction B comprising:
40.1 percent by weight of water
41.8 percent by weight of NMEDA
18.1 percent by weight of EDA
was withdrawn as a side draw between stages 17 and 18.

Accordingly, the weight ratio of water to NMEDA was approximately 0.96:1.

1847,93 kg/h of fraction C comprising.

| | |
|---|---|
| water: | 1,05 percent by weight |
| NMEDA: | 0,01 percent by weight |
| EDA: | 32,40 percent by weight |
| PIP: | 2,30 percent by weight |
| MEOA: | 18,55 percent by weight |
| MEG: | 41,11 percent by weight |
| DETA: | 1.88 percent by weight |
| AEPIP: | 0.20 percent by weight |
| AEEA: | 1.57 percent by weight |

was with withdrawn from the bottoms of the NMFDA Removal Column and fed to an additional Residual Water Removal Column. The weight ratio of EDA to water in fraction C was approximately 30.9 to 1.

Fraction C was further separated into a fraction C-1 and into a fraction C-2 in a Residual Water Removal Column equipped with a reboiler and having 36 stages.

Fraction C was introduced above stage 36.

The Residual Water Removal Column was operated at a bottom temperature of 181°C and a top pressure of 1.3 bar.

395,93 kg/h or fraction C-1 comprising:

| | |
|---|---|
| water: | 4.86 percent by weight |
| NMEDA: | 0.028 percent by weight |
| EDA: | 69.36 percent by weight |
| PIP: | 2.88 percent by weight |
| MEOA: | 13.91 percent by weight |
| MEG: | 8.55 percent by weight |
| DETA: | 0.24 percent by weight |
| AEPIP: | 0.03 percent by weight |
| AEEA: | 0.12 percent by weight |

was withdrawn from the top of the Residual Water Removal Column. This stream was directly fed in vaporous form without condensation to the bottom of the NMEDA Removal Column.

1452,00 kg/h of a fraction C-2 comprising:

| | |
|---|---|
| water: | 0.01 percent by weight |
| NMEDA: | 0.00009 percent by weight |
| EDA: | 22.32 percent by weight |
| PIP: | 2,15 percent by weight |
| MEOA: | 19.80 percent by weight |
| MEG: | 49.99 percent by weight |
| DETA: | 2.33 percent by weight |
| AEPIP: | 0.25 percent by weight |
| AEEA: | 1.97 percent by weight |

was withdrawn from the bottom of the Residual Water Removal Column and was purified in a dividing wall column. A pure EDA fraction comprising 500 ppm by weight of water and 2 ppm by weight of NMEDA was obtained.

### Example 2: Provision of a feed comprising EDA, NMEDA and water

A feed was prepared in a combined MEG/MEOA process.

After the removal of and hydrogen and ammonia the feed comprised:

| | |
|---|---|
| water: | 9.03 percent by weight |
| NMEDA: | 0.07 percent by weight |
| EDA: | 7.07 percent by weight |
| PIP: | 0.72 percent by weight |
| MEOA: | 12.96 percent by weight |
| MEG: | 68.82 percent by weight |
| DETA: | 0.72 percent by weight |
| AEEA: | 0.07 percent by weight |
| TETA: | 0.51 percent by weight |

The molar ratio of MEG to EDA in the feed stream is 9.43 to 1. The molar ratio of hydroxyl groups to EDA was approximate 18.9:1.

2459,7 kg/h of the feed stream were fed to an NMEDA Removal Column where the feed was separated into a fraction A, a fraction B, and a fraction C.

The NMEDA-Removal Column was equipped with a reboiler and had 25 stages The feed was introduced above stage 12 (counted from the bottom).

The bottom temperature was 180°C.

354,15 kg/g of fraction A comprising water and 100 weight ppm NMEDA (weight ratio of water to NMEDA: approx. 10000:1) was withdrawn from the top of the column and fed to a condenser operated at 100°C .

134,79 kg/h of the condensed stream were refluxed back to the top of the NMEDA Removal Column, and a stream of 219,36 kg/h was removed.

A fraction A comprising water and 100 weight ppm NMEDA was removed from the top of the column. Accordingly, the weight ratio of water to NMEDA was approx. 10000:1.

### 4,35 kg/h of a fraction B comprising:

5.43 percent by weight of water
36.9 percent by weight of NMEDA
7.62 percent by weight of EDA
was withdrawn as a side draw between stages 16 and 17.

Accordingly, the weight ratio of water to NMEDA was approximately 6.17:1.

2235.92 kg/h of fraction C comprising.

| | |
|---|---|
| water: | 1,05 percent by weight |
| NMEDA: | 0,002 percent by weight |
| EDA: | 7.76 percent by weight |
| PIP: | 0.79 percent by weight |
| MEOA: | 14.26 percent by weight |
| MEG: | 75.71 percent by weight |
| DETA: | 0.79 percent by weight |
| AEEA: | 0.04 percent by weight |
| TETA: | 0.56 percent by weight |

was with withdrawn from the bottoms of the NMEDA Removal Column. The weight ratio of EDA to water in fraction C was approximately 236.5 to 1.

Fraction C was purified in a distillation column with 60 stages. Fraction C was fed to the column between stage 36 and 37 (counted from the bottom). A top fraction comprising 99.80 percent by weight of EDA, 0.12 percent by weight of water. 0.03 percent by weight of NMEDA and 0.05 percent by weight of PIP was obtained.

Example 1 shows that by separating the feed stream from an EDA preparation process into three fractions according to the present invention from the NMEDA Removal Column, a commercial grade of EDA can be obtained in only one additional distillation step. The losses of EDA are comparatively small and lie in the same order of magnitude as the NMEDA present in the feed.

In Example 2, the water concentration in the bottom of the NMEDA Removal Column is comparatively higher. This allows to obtain an EDA having an ultra-low NMEDA content.

## Claims

1. A method for the manufacture of ethyleneamine from a mixture comprising water (H₂O), ethylenediamine (EDA) and N-methylethylenediame (NMEDA), comprising the steps of:
(i) providing a feed stream comprising EDA, NMEDA and water;
(ii) separating the feed stream provided in step (i) in the one or more distillation columns into
a. a fraction A comprising water and NMEDA wherein the weight ratio of water to NMEDA in fraction A is more than 100:1;
b. a fraction B comprising water, NMEDA and EDA wherein the weight ratio of water to NMEDA is in the range of 1:100 to 100:1; and
c. a fraction C comprising water and EDA wherein the weight ration of EDA to water is more than 5:1, and wherein the water content in fraction C is in the range of 0.1 to 10 percent by weight;
(iii) separating fraction C into:
- a fraction C-1 comprising EDA, NMEDA and water, wherein the weight ratio of EDA to water is in the range of 1:5 to 5:1 and the weight ratio of NMEDA to EDA is in the range of 100:1 to 1:1, and
- a fraction C-2 comprising EDA and preferably components with a boiling point higher than EDA and in which the NMEDA content is 0.01 percent by weight or less.

2. A method according to claim 1, wherein the feed comprising EDA, NMEDA and water comprises 1 to 30 percent by weight of EDA, 1 to 30 percent by weight of water and 0.001 to 1 percent by weight of NMEDA.

3. A method according to at least one of claims 1 to 2, wherein the feed provided in step (i) additionally comprises components having a boiling point higher than EDA.

4. A method according to claim 3 wherein one of the components having a boiling point higher than EDA is monoethanolamine (MEOA).

5. A method according to at least one of claims 1 to 4, wherein providing a feed in step (i) comprises the steps of:
(i-a) carrying out an EDA preparation process to obtain an output comprising EDA, NMEDA and water,
(i-b) removing ammonia and/or hydrogen from the output produced in step (i-a); and
(i-c) optionally adding an additional adjuvant.

6. A method according to claim 5, wherein the EDA preparation process in step (i-a) is a process in which monoethylene glycol (MEG) is converted with ammonia in the presence of an amination catalyst and hydrogen.

7. A method according to at least one of claims 1 to 6, wherein the separation step (ii) is carried out under conditions in which the azeotrope between EDA and water is broken, impaired or otherwise modified so that fraction A can be separated from NMEDA as a low boiling fraction.

8. A method according to at least one of claim 7, wherein the separation (ii) is carried out at a pressure lower than that required to break the EDA-water azeotrope.

9. A method according to claim 8, wherein the feed provided in step (i) comprises an additional adjuvant

10. A method according to claim 9, wherein the additional adjuvant is a compound other than an ethanolamine or an ethyleneamine.

11. A method according to claim 10, wherein the additional adjuvant is a hydroxyl group comprising compound.

12. A method according to claim 11, wherein the additional adjuvant) is selected from the group of consisting of:
(i) aliphatic monools,
(ii) aliphatic diols;
(iii) aliphatic triols; and
(iv) aliphatic tetrols.

13. A method according to claim 12, wherein the additional adjuvant provided in step (i) is an aliphatic diol selected from the group of ethylene glycol, 1,2 propylene glycol and 1,2 butanediol.

14. A method according to at least one of claims 11 to 13, wherein amount of additional adjuvant provided in step (i)is adjusted so that the molar ratio of hydroxyl groups present in the feed to EDA molecules present in the feed is 1:1 or more.

15. A method according to claim 13, wherein the additional adjuvant is MEG.

16. A method according to claim 15, wherein the molar ratio of MEG to EDA is 1:1 or more.

17. A method according to a least one claims 1 to 16 wherein the separation in step (ii) is carried out in a one distillation column in which fraction A is drawn-off at the top, fraction B is drawn-off as a side fraction and fraction C is drawn-off at the bottom of the distillation column.

18. A method according to claim 17, wherein the bottom temperature of the column is 160°C or less.

19. A method according to at least one of claims 1 to 18, wherein the fraction C-2 is further separated into:
- a fraction D-1 comprising EDA,
- a fraction D-2 comprising PIP and
- a fraction D-3, comprising components having a boiling point higher than PIP, in particularly MEOA, MEG, DETA, AEE, DEOA and TETA.

20. A method according to claim 19, wherein the separation of fraction C-2 is conducted in a single dividing wall column.

## Patentansprüche

1. Ein Verfahren zur Herstellung von Ethylenamin aus einem Gemisch, das Wasser (H₂O), Ethylendiamin (EDA) und N-Methylethylendiamin
(NMEDA) umfasst, mit den Schritten:
(i) Bereitstellen eines Einspeisestroms, der EDA, NMEDA und Wasser umfasst;
(ii) Trennen des in Schritt (i) bereitgestellten Einspeisestroms in einer oder mehreren Destillationskolonnen in
a. eine Fraktion A, die Wasser und NMEDA umfasst, wobei das Gewichtsverhältnis von Wasser zu NMEDA in Fraktion A mehr als 100:1 beträgt;
b. eine Fraktion B, die Wasser, NMEDA und EDA umfasst, wobei das Gewichtsverhältnis von Was-
ser zu NMEDA im Bereich von 1:100 bis 100:1 liegt; und
c. eine Fraktion C, die Wasser und EDA umfasst, wobei das Gewichtsverhältnis von EDA zu Was-
ser mehr als 5:1 beträgt und wobei der Wassergehalt in Fraktion C im Bereich von 0,1 bis 10 Gewichtsprozent liegt;
(iii) Trennen von Fraktion C in:
eine Fraktion C-1, die EDA, NMEDA und Wasser umfasst, wobei das Gewichtsverhältnis von EDA zu
Wasser im Bereich von 1:5 bis 5:1 liegt und das Gewichtsverhältnis von NMEDA zu EDA im Bereich
von 100:1 bis 1:1 liegt, und
eine Fraktion C-2, die EDA und vorzugsweise Komponenten mit einem höheren Siedepunkt als EDA umfasst und in der der NMEDA-Gehalt 0,01 Gewichtsprozent oder
weniger beträgt.

2. Verfahren nach Anspruch 1, wobei der Einsatz, der EDA, NMEDA und Wasser umfasst,
1 bis 30 Gewichtsprozent EDA, 1 bis 30 Gewichtsprozent Wasser und 0,001 bis 1 Gewichtsprozent NMEDA umfasst.

3. Verfahren nach mindestens einem der Ansprüche 1 bis 2, wobei der in Schritt (i) bereitgestellte Einsatz zusätzlich Komponenten mit einem höheren Siedepunkt als EDA umfasst.

4. Verfahren nach Anspruch 3, wobei eine der Komponenten mit einem höheren Siedepunkt als EDA Monoethanolamin (MEOA) ist.

5. Verfahren nach mindestens einem der Ansprüche 1 bis 4, wobei das Bereitstellen eines Einsatzes in Schritt (i) die folgenden Schritte umfasst:
(i-a) Durchführen eines EDA-Herstellungsverfahrens, um ein Produkt zu erhalten, das EDA, NMEDA und Wasser umfasst,
(i-b) Entfernen von Ammoniak und/oder Wasserstoff aus dem in Schritt (i-a) erzeugten Produkt; und (i-c) optionales Hinzufügen eines zusätzlichen Hilfsstoffs.

6. Verfahren nach Anspruch 5, wobei das EDA-Herstellungsverfahren in Schritt (i-a) ein Verfahren ist, bei dem Monoethylenglykol
(MEG) mit Ammoniak in Gegenwart eines
Aminierungskatalysators und Wasserstoff
umgesetzt wird.

7. Verfahren nach mindestens einem der Ansprüche 1 bis 6, wobei der Trennschritt (ii)
unter Bedingungen durchgeführt wird, unter denen das Azeotrop zwischen EDA und Wasser gebrochen, beeinträchtigt oder anderweitig modifiziert wird, sodass Fraktion A von NMEDA als niedrigsiedende Fraktion abgetrennt werden kann.

8. Verfahren nach mindestens einem der Ansprüche 7, wobei die Trennung (ii) bei
einem Druck durchgeführt wird, der niedriger ist als der Druck, der erforderlich ist, um das EDA-Wasser-Azeotrop zu brechen.

9. Verfahren nach Anspruch 8, wobei der in Schritt (i) bereitgestellte Einsatz einen zusätzlichen Hilfsstoff umfasst

10. Verfahren nach Anspruch 9, wobei der zusätzliche Hilfsstoff eine Verbindung ist, die weder ein Ethanolamin noch ein Ethylenamin ist.

11. Verfahren nach Anspruch 10, wobei der zusätzliche Hilfsstoff eine Hydroxylgruppen enthaltende Verbindung ist.

12. Verfahren nach Anspruch 11, wobei der zusätzliche Hilfsstoff aus der Gruppe ausgewählt wird, die besteht aus:
(i) aliphatischen Monolen,
(ii) aliphatischen Diolen;
(iii) aliphatischen Triolen; und
(iv) aliphatischen Tetrolen.

13. Verfahren nach Anspruch 12, wobei der in Schritt (i) bereitgestellte zusätzliche Hilfsstoff ein aliphatisches Diol ist, das aus der Gruppe von Ethylenglykol, 1,2-Propylenglykol und 1,2-Butandiol ausgewählt wird

14. Verfahren nach mindestens einem der Ansprüche 11 bis 13, wobei die Menge des in Schritt (i) bereitgestellten zusätzlichen Hilfsstoffs so eingestellt wird, dass das Molverhältnis der im Einsatz vorhandenen Hydroxylgruppen zu den im Einsatz vorhandenen EDA-Molekülen 1:1 oder mehr beträgt.

15. Verfahren nach Anspruch 13, wobei der zusätzliche Hilfsstoff MEG ist.

16. Verfahren nach Anspruch 15, wobei das Molverhältnis von MEG zu EDA 1:1 oder mehr beträgt.

17. Verfahren nach mindestens einem der Ansprüche 1 bis 16, wobei die Trennung in Schritt (ii) in einer Destillationskolonne durchgeführt wird, in der Fraktion A am Kopf abgezogen wird, Fraktion B als Seitenfraktion abgezogen wird und Fraktion C am Boden der Destillationskolonne abgezogen wird.

18. Verfahren nach Anspruch 17, wobei die Bodentemperatur der Kolonne 160 °C oder weniger beträgt.

19. Verfahren nach mindestens einem der Ansprüche 1 bis 18, wobei die Fraktion C-2 weiter getrennt wird in:
eine Fraktion D-1, die EDA umfasst,
eine Fraktion D-2, die PIP umfasst, und
eine Fraktion D-3, die Komponenten mit einem höheren Siedepunkt als PIP umfasst, insbesondere MEOA, MEG, DETA, AEE, DEOA und TETA.

20. Verfahren nach Anspruch 19, wobei die Trennung von Fraktion C-2 in einer einzigen Trennwandkolonne durchgeführt wird.

## Revendications

1. Un procédé pour la fabrication d'éthylènediamine à partir d'un mélange comprenant de l'eau (H₂O), de l'éthylènediamine (EDA) et de la N-méthyléthylènediamine
(NMEDA), comprenant les étapes suivantes :
(i) fournir un flux d'alimentation comprenant de l'EDA, de la NMEDA et de l'eau ;
(ii) séparer le flux d'alimentation fourni à l'étape (i) dans une ou plusieurs colonnes de distillation en
a. une fraction A comprenant de l'eau et de la NMEDA, dans laquelle le rapport en poids de l'eau à la NMEDA dans la fraction A est supérieur à 100:1 ;
b. une fraction B comprenant de l'eau, de la NMEDA et de l'EDA, dans laquelle le rapport en poids de l'eau
à la NMEDA est compris entre 1:100 et 100:1 ; et
c. une fraction C comprenant de l'eau et de l'EDA, dans laquelle le rapport en poids de l'EDA à l'eau est supérieur à 5:1, et dans laquelle la teneur en eau dans la fraction C est comprise entre 0,1 et 10 pour cent en poids ;
(iii) séparer la fraction C en :
une fraction C-1 comprenant de l'EDA, de la NMEDA et de l'eau, dans laquelle le rapport en poids de l'EDA à
l'eau est compris entre 1:5 et 5:1 et le rapport en poids de la NMEDA à l'EDA est compris entre 100:1 et 1:1, et
une fraction C-2 comprenant de l'EDA et de préférence des composants ayant un point d'ébullition supérieur à celui de l'EDA et dans laquelle la teneur en NMEDA est de 0,01 pour cent en poids ou moins.

2. Un procédé selon la revendication 1, dans lequel l'alimentation comprenant de l'EDA, de la NMEDA et de l'eau
comprend 1 à 30 pour cent en poids d'EDA, 1 à 30 pour cent en poids d'eau et 0,001 à 1 pour cent en poids de NMEDA.

3. Un procédé selon au moins l'une des revendications 1 à 2, dans lequel l'alimentation fournie à l'étape (i)
comprend en outre des composants ayant un point d'ébullition supérieur à celui de l'EDA.

4. Un procédé selon la revendication 3, dans lequel l'un des composants ayant un point d'ébullition supérieur à celui de l'EDA est la monoéthanolamine (MEOA).

5. Un procédé selon au moins l'une des revendications 1 à 4, dans lequel la fourniture d'une alimentation à l'étape (i) comprend les étapes suivantes :
(i-a) réaliser un procédé de préparation d'EDA pour obtenir un produit comprenant de l'EDA, de la NMEDA et de l'eau,
(i-b) éliminer l'ammoniac et/ou l'hydrogène du produit obtenu à l'étape (i-a) ; et (i-c) ajouter éventuellement un adjuvant supplémentaire.

6. Un procédé selon la revendication 5, dans lequel le procédé de préparation d'EDA à l'étape (i-a) est un procédé dans lequel le monoéthylène glycol
(MEG) est converti avec de l'ammoniac en
présence d'un catalyseur d'amination et
d'hydrogène.

7. Un procédé selon au moins l'une des revendications 1 à 6, dans lequel l'étape de séparation (ii) est réalisée dans des conditions dans lesquelles l'azéotrope entre l'EDA et l'eau est rompu, altéré ou autrement modifié de sorte que la fraction A peut être séparée de la NMEDA en tant que fraction à bas point d'ébullition.

8. Un procédé selon au moins l'une des revendications 7, dans lequel la séparation (ii) est réalisée à une pression inférieure à celle requise pour rompre l'azéotrope EDA-eau.

9. Un procédé selon la revendication 8, dans lequel l'alimentation fournie à l'étape (i) comprend un adjuvant supplémentaire

10. Un procédé selon la revendication 9, dans lequel l'adjuvant supplémentaire est un composé autre qu'une éthanolamine ou une éthylènediamine.

11. Un procédé selon la revendication 10, dans lequel l'adjuvant supplémentaire est un composé comprenant
un groupe hydroxyle.

12. Un procédé selon la revendication 11, dans lequel l'adjuvant supplémentaire est sélectionné dans le groupe constitué de :
(i) monools aliphatiques,
(ii) diols aliphatiques ;
(iii) triols aliphatiques ; et
(iv) tétrols aliphatiques.

13. Un procédé selon la revendication 12, dans lequel l'adjuvant supplémentaire fourni à l'étape (i) est un diol aliphatique sélectionné dans le groupe comprenant l'éthylène glycol, le 1,2-propylène glycol et le 1,2-bu-
tanediol

14. Un procédé selon au moins l'une des revendications 11 à 13, dans lequel la quantité d'adjuvant supplémentaire fournie à l'étape (i) est ajustée de sorte que le rapport molaire des groupes hydroxyle présents dans l'alimentation aux molécules d'EDA présentes dans l'alimentation est de 1:1 ou plus.

15. Un procédé selon la revendication 13, dans lequel l'adjuvant supplémentaire est le MEG.

16. Un procédé selon la revendication 15, dans lequel le rapport molaire du MEG à l'EDA est de 1:1 ou plus.

17. Un procédé selon au moins l'une des revendications 1 à 16, dans lequel la séparation à l'étape (ii) est réalisée dans une colonne de distillation dans laquelle la fraction A est soutirée en tête, la fraction B est soutirée en tant que fraction latérale et la fraction C est soutirée en bas de la colonne de distillation.

18. Un procédé selon la revendication 17, dans lequel la température de fond de la colonne est de 160°C ou moins.

19. Un procédé selon au moins l'une des revendications 1 à 18, dans lequel la fraction C-2 est en outre
séparée en :
une fraction D-1 comprenant de l'EDA,
une fraction D-2 comprenant du PIP et
une fraction D-3, comprenant des composants ayant un point d'ébullition supérieur à celui du PIP, en particulier la MEOA, le MEG, la DETA, l'AEE, la DEOA et la TETA.

20. Un procédé selon la revendication 19, dans lequel la séparation de la fraction C-2 est réalisée dans une colonne à paroi de séparation unique.
